(19) Europäisches Patentamt
European Patent Office
Office européen des brevets

(11) Veröffentlichungsnummer: **0 086 324**
**B1**

(12) # EUROPÄISCHE PATENTSCHRIFT

(45) Veröffentlichungstag der Patentschrift: **21.09.88**

(51) Int. Cl.⁴: **C 07 D 307/62**

(21) Anmeldenummer: **83100116.9**

(22) Anmeldetag: **08.01.83**

(54) **Verfahren zur Herstellung von Ascorbinsäure.**

(30) Priorität: **12.02.82 CH 891/82**
**10.12.82 CH 7218/82**

(43) Veröffentlichungstag der Anmeldung: **24.08.83 Patentblatt 83/34**

(45) Bekanntmachung des Hinweises auf die Patenterteilung: **21.09.88 Patentblatt 88/38**

(84) Benannte Vertragsstaaten: **AT BE CH DE FR GB IT LI NL**

(56) Entgegenhaltungen: **CH-A-256 514**

(73) Patentinhaber: **F. HOFFMANN- LA ROCHE & CO. Aktiengesellschaft, CH- 4002 Basel (CH)**

(72) Erfinder: **Fahrni, Peter, Dr., Obere Hofackerstrasse 23, CH- 4414 Füllinsdorf (CH)**
Erfinder: **Siegfried, Theodor, Dr., Türkheimerstrasse 5, CH- 4009 Basel (CH)**

(74) Vertreter: **Cottong, Norbert A., Grenzacherstrasse 124 Postfach 3255, CH- 4002 Basel (CH)**

**EP 0 086 324 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft ein neues Verfahren zur Herstellung von Ascorbinsäure aus 2-Ketogulonsäureestern (KGSE).

Ketogulonsäure oder deren Ester, welche in herkömmlichen Ascorbinsäuresynthesen in der vorletzten Stufe anfallen, müssen zur Ascorbinsäure umgelagert werden. Bei dieser Umlagerung, welche unter sauren oder alkalischen Bedingungen durchgeführt werden kann, ergeben sich jeweils gewisse Probleme. So besteht einerseits bei der sauren Umlagerung das Problem insbesondere darin, daß die Ausbeuten gewöhnlich nicht befriedigend sind und die Qualität der erhaltenen Ascorbinsäure zu wünschen übrig läßt. Zudem sind die Reaktionszeiten bei der sauren Umlagerung im allgemeinen sehr lang.

Bei der alkalischen Umlagerung andererseits besteht das Problem im wesentlichen darin, daß die zur Umlagerung verwendeten Basen mit der Ascorbinsäure Salze bilden, welche zwecks Isolierung der reinen Ascorbinsäure in der Regel durch Zugabe einer Säure wieder gespalten werden müssen. Hierbei entstehen nun wiederum Salze, von welchen die Ascorbinsäure nur schwer abgetrennt werden kann und welche mindestens zum Teil nicht weiter verwendbar sind; siehe diesbezüglich auch A. Grüssner und W. Schlegel, Wissenschaftliche Veröffentlichungen der Deutschen Gesellschaft für Ernährung, Band 14, Dr. Dietrich Steinkopff Verlag, Darmstadt (1965), Seiten 1 - 16, DDR Patentschrift Nr. 86 627 (20.12.1971), Französische Patentschrift Nr. 779 883 (13.04.1935), Schweizerische Patentschrift Nr. 256 514 (01.03.1949).

Es bestand somit ein Bedürfnis nach einem Verfahren, gemäß welchem Ascorbinsäure in einfacher Weise aus Ketogulonsäureestern und in guter Ausbeute erhalten werden kann und bei welchem die zur Umlagerung benötigte Base leicht zurückgewonnen und in den Prozeß zurückgeführt werden kann.

Die geschilderten Probleme konnten nun mittels des erfindungsgemäßen Verfahrens behoben werden. Es hat sich nämlich herausgestellt, daß bei Verwendung von gewissen Aminen bei der Umlagerung von KGSE die geschilderten Probleme nicht auftreten.

Das erfindungsgemäße Verfahren ist demnach dadurch gekennzeichnet, daß man einen Ketogulonsäureester der allgemeinen Formel

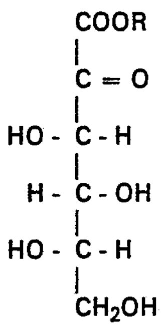

I

worin R $C_{1-5}$-Alkyl bedeutet,

mit einem Amin mit 12 bis 38 Kohlenstoffatomen in einem protischen oder aprotisch-dipolaren organischen Lösungsmittel umsetzt und daß man das erhaltene Ascorbinsäure-Aminsalz der allgemeinen Formel

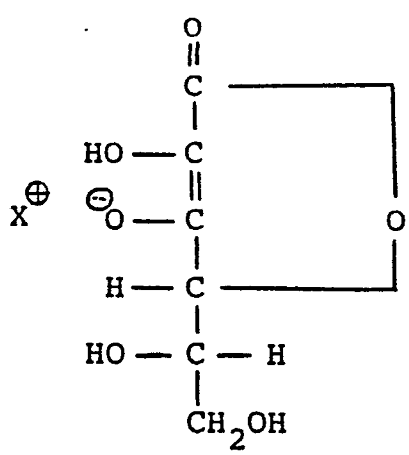

II

worin $X^{\oplus}$ das Ammoniumion des eingesetzten Amins darstellt,

ohne Neutralisation spaltet und die Ascorbinsäure sowie gegebenenfalls das eingesetzte Amin isoliert, wobei die Isolierung der ersteren durch flüßig-flüßig Extraktion oder durch Digerieren in einem unpolaren organischen Lösungsmittel erfolgt.

Der Ausdruck "$C_{1-5}$-Alkyl" bedeutet im Rahmen der vorliegenden Erfindung Alkylgruppen mit 1 bis 3

Kohlenstoffatomen wie Methyl, Äthyl, Propyl, Isopropyl und Butyl. Bevorzugt sind Alkylgruppen mit 1 bis 3 Kohlenstoffatomen und insbesondere Methyl und Äthyl.

Als Amine kommen im Rahmen der vorliegenden Erfindung primäre, sekundäre und tertiäre Amine mit 12 bis 38 Kohlenstoffatomen in Betracht. Die untere Grenze der Kohlenstoffanzahl ist u.a. durch die Löslichkeit in dem zur Spaltung des Ascorbinsäure-Aminsalzes der Formel II verwendeten organischen Lösungsmittel gegeben. Die Obergrenze der Kohlenstoffatomanzahl wiederum ist gegeben durch die Löslichkeit in dem für die Reaktion zwischen dem KGSE und dem Amin verwendeten Lösungsmittel.

Die verwendbaren Amine sind in der Reihenfolge bevorzugt:

tert. Amine > sek. Amine > prim. Amine

Primäre Amine umfassen sowohl geradkettige, als auch verzweigte Amine, wobei die verzweigten bevorzugt sind. Besonders bevorzugt sind zudem solche mit 12 bis 24 Kohlenstoffatomen.

Unter primären Aminen sind zudem gewisse flüßige, basische Ionentauscher wie etwa Primene® JMT oder auch Amberlite® LA-3 zu verstehen.

Sekundäre Amine umfassen sowohl geradkettige, als auch verzweigte Alkylamine, sowie aromatische Amine. Bevorzugte sekundäre Amine sind verzweigte Alkylamine und insbesondere solche mit 18 bis 25 Kohlenstoffatomen. Von den aromatischen sekundären Aminen ist das Dibenzylamin bevorzugt. Unter sekundären Aminen sind zudem gewiße flüssige, basische Ionentauscher wie etwa Amberlite® LA-1 oder Amberlite® LA-2 zu verstehen.

Tertiäre Amine umfassen insbesondere geradkettige und teilweise verzweigte aliphatische Amine, wobei die geradkettigen Alkylamine und insbesondere solche mit 15 bis 30 Kohlenstoffatomen bevorzugt sind.

Sowohl bei den sekundären, als auch bei den tertiären Aminen kommen sowohl solche mit gleichen, als auch solche mit verschiedenen Alkylketten in Frage.

Als Beispiele von im Rahmen der vorliegenden Erfindung in Frage kommenden und zudem bevorzugten Aminen können folgende genannt werden:

Primene® JMT
Amberlite® LA-3
α-Aminodiphenylmethan
1,2-Diphenyläthylamin
Bis-(2-äthyl-hexyl)-amin
Amberlite® LA-1
Amberlite® LA-2
Dibenzylamin
Dioctylamin
Tripentylamin
Triisopentylamin
N,N-Dioctylmethylamin
Trihexylamin
Triheptylamin
Trioctylamin
Tridodecylamin

Die Umsetzung der Ketogulonsäureester der Formel I, welche im übrigen auch in pyranoider oder furanoider Form vorliegen können, mit einem Amin erfolgt in einem geeigneten, weiter unter definierten organischen Lösungsmittel, bzw. in einem Lösungsmittelgemisch, in welchem der KGSE und/oder das verwendete Amin zumindest teilweise löslich sind. In Frage kommen hierbei erfindungsgemäß sowohl protische, als auch aprotische-dipolare Lösungsmittel. Von den protischen können beispielsweise genannt werden niedere Alkohole mit 1 bis 5 Kohlenstoffatomen, wie Methanol, Äthanol, Propanol, Isopropanol. Als Beispiele von aprotisch-dipolaren Lösungsmitteln können genannt werden Acetonitril, Dimethylformamid, Dioxan, Monoglyme, Methylcellosolve (Äthylenglycolmonomethyläther). Bevorzugte Lösungsmittel sind die Alkohole und insbesondere Methanol. Die als Ausgangsmaterial verwendeten KGSE der Formel I sind bekannte oder Analoge bekannter Verbindungen. Sie können als solche in der Reaktion eingesetzt werden oder auch vorgängig in situ aus 2-Ketogulonsäure durch Veresterung mit einem entsprechenden Alkohol hergestellt werden.

Die verwendete Menge an Lösungsmittel ist an und für sich nicht kritisch, liegt aber zweckmäßig bei einem Verhältnis von 1 : 1 bis 10 : 1 (ml/g), bezogen auf den KGSE und das eingesetzte Amin.

Die in dem erfindungsgemäßen Verfahren verwendete Menge Amin beträgt zweckmäßig von 0,1 bis 1,5, vorzugsweise von 0,5 bis 1,1 und insbesondere von 0,9 bis 1 Mol pro Mol Ketogulonsäureester.

Die Temperatur, der Druck und die Reaktionsdauer sind als solche keine kritischen Größen in dem erfindungsgemäßen Verfahren.

Die Temperatur ist der Geschwindigkeits-bestimmende Faktor. Die obere Grenze ist dabei durch die Stabilität der Reaktionspartner gegeben. Zweckmäßig erfolgt die Reaktion bei einer Temperatur bis 90° C, vorzugsweise von 50° C bis 75° C und insbesondere bei 65° C.

Der Druck ist bei dem erfindungsgemäßen Verfahren keine kritische Größe, so daß dieses ohne weiteres bei

Normaldruck durchgeführt werden kann. Es kann jedoch u.U. auch unter erhöhtem Druck gearbeitet werden, bei entsprechend erhöhter Temperatur und entsprechend geringeren Reaktionszeiten.

Die Reaktionszeit ist abhängig von der Reaktionstemperatur und liegt im Hinblick auf die optimalen Temperaturen zwischen 2 bis 7 und insbesondere zwischen 3 bis 5 Stunden.

Die Umsetzung des KGSE und des Amins kann sowohl in Anwesenheit, als auch in Abwesenheit von Luft durchgeführt werden. Vorzugsweise wird allerdings unter Luftausschluß, d.h. unter Inertgas, wie etwa Stickstoff, Argon gearbeitet.

Die Spaltung des erhaltenen Ascorbinsäure-Aminsalzes erfolgt erfindungsgemäß ohne Neutralisation, d.h. ohne Zugabe einer Säure oder einer Base. Die Spaltung, sowie die Isolierung der reinen Ascorbinsäure und gegebenenfalls des eingesetzten Amins, erfolgt durch flüßig-flüßig Extraktion, wobei die freie Ascorbinsäure in die polare und das freie Amin in die unpolare Phase übergehen, oder u.U. durch bloßes Digerieren, d.h. Erhitzen mit einem geeigneten organischen Lösungsmittel.

Für die Extraktion geeignete unpolare Lösungsmittel sind solche, in denen das verwendete Amin gut löslich ist. Zweckmäßig verwendet man ein aprotisch apolares Lösungsmittel, wie aliphatische oder aromatische Kohlenwasserstoffe, z. B. Hexan, Petroläther, Benzol, Toluol, Xylol und dergleichen. Geeignete polare Lösungsmittel bzw. Lösungsmittelgemische sind solche, in denen die Ascorbinsäure gut löslich ist und welche mit den vorhergehend erwähnten unpolaren Lösungsmitteln nicht mischbar sind. Zweckmäßig verwendet man Wasser, Methanol, Äthanol, Acetonitril, Aceton bzw. Gemische hiervon. Als besonders zweckmäßig hat sich die Anwesenheit zumindest einer geringen Menge Wasser erwiesen. Besonders bevorzugt ist Wasser oder ein Wasser-Methanol-Gemisch. Die Temperatur, bei welcher die Extraktion durchgeführt wird, ist an und für sich nicht kritisch, und es kann sowohl bei Raumtemperatur, als auch bei erhöhter Temperatur gearbeitet werden. Vorzugsweise wird die Extraktion bei etwa Raumtemperatur durchgeführt. Nach erfolgter Extraktion können die Ascorbinsäure sowie das verwendete Amin aus den jeweiligen sie enthaltenden Phasen in an sich bekannter Weise leicht isoliert werden.

Für das Digerieren sind im Prinzip die gleichen unpolaren Lösungsmittel geeignet wie vorhergehend für die Extraktion angegeben. Das Erhitzen erfolgt zweckmäßig bei einer Temperatur von 50° C bis Rückflußtemperatur des verwendeten Lösungsmittels. Vorzugsweise erfolgt das Digerieren bei Rückflußtemperatur. Bei diesem Digerieren wird das Ascorbinsäure-Aminsalz zerlegt, wobei die reine Ascorbinsäure ausfällt und das Amin in die organische Phase übergeht, aus welcher es dann leicht in an sich bekannter Weise isoliert werden kann.

Sämtliche vorhergehend erwähnten Operationen können im übrigen sowohl kontinuierlich, als auch batchwise durchgeführt werden.

**Beispiel 1**

In einen 500-ml-Sulfierkolben, versehen mit Rührer, Thermometer, Rückflußkühler und 50-ml-Tropftrichter, werden unter Argon 26,8 g (128,7 mMol) Ketogulonsäuremethylester und 125 ml Methanol gegeben. Hierauf wird das Gemisch unter Rühren auf 65°C (Rückfluß) erwärmt, wobei sich der Ester löst. Mittels des Tropftrichters werden dann 38 g (109 Mol-%) Trihexylamin (99,9 %-ig) innert 15 Minuten zugetropft. Gegen Ende der Trihexylamin-Zugabe wird das Reaktionsgemisch leicht trübe, 5 Minuten später klärt sich die Lösung jedoch wieder. Nun wird während 4 3/4 Stunden bei Rückfluß weitergerührt, wobei sich das Reaktionsgemisch gelb färbt. Nach insgesamt 5 Stunden wird das Gemisch mit Methanol in einen 500-ml-Rundkolben gespült und am Rollverdampfer bei 45° C/20 mbar eingeengt. Als Rückstand erhält man ein gelbes, viskoses Öl.

Das vorhergehend erhaltene Öl wird in 50 ml entionisiertem Wasser gelöst und mit ca. 20 ml Wasser in einen mit Stickstoff begasten Rotations-Perforator gespült. Hierauf wird das Gemisch mit etwa 500 ml n-Hexan während 20 Stunden kontinuierlich extrahiert. Die Wasserphase wird dabei fast farblos, die organische Phase gelb. Nach beendeter Extraktion werden die beiden im Extraktor verbleibenden Phasen in einem Scheidetrichter getrennt. Die organischen Phasen werden vereinigt, über etwa 30g $Na_2SO_4$ getrocknet und am Rollverdampfer bei 45° C/100 - 20 mbar eingedampft. Zurück bleiben 37,8 g gelbes Trihexylamin mit einem Gehalt von 97,6 %. Dies entspricht einer Ausbeute an Trihexylamin von 97,2 %.

Die nach der Extraktion des Trihexylamins angefallene Wasserphase enthält 21,9 g Ascorbinsäure, entsprechend einer Ausbeute von 96,4 %.

**Beispiel 2**

In zu Beispiel 1 analoger Weise wurde die Umsetzung des Ketogulonsäuremethylesters mit dem Trihexylamin in weiteren Lösungsmittelndurchgeführt. Die Resultate sind in der folgenden Tabelle zusammengestellt.

**Tabelle**

| Lösungsmittel | Ausbeute an Ascorbinsäure | Zurückgewonnenes Amin |
|---|---|---|
| Äthanol | 92 % | 98,6 % |
| Isopropanol | 94,1 % | 97,9 % |
| Methylcollosolve | 93,5 % | 97,9 % |
| Acetonitril | 95,8 % | 91,3 % |

**Beispiel 3**

In einen 500-ml-Sulfierkolben, versehen mit Rührer, Thermometer und Rückflußkühler, werden unter Argon 26,8 g (128,7 mMol) Ketogulonsäuremethylester und 125 ml Methanol gegeben. Hierauf wird das Gemisch unter Rühren auf 65°C (Rückfluß) erwärmt, wobei sich der Ester löst. Dann werden 17,5 g (50 Mol-%) Trihexylamin (99,9 %-ig) innert 15 Minuten zugegeben. Nun wird während 24 Stunden bei Rückfluß weitergerührt, wobei sich das Reaktionsgemisch gelb färbt. Anschließend wird das Gemisch mit Methanol in einen 500-ml-Rundkolben gespült und am Rollverdampfer bei 45°C/20 mbar eingeengt. Als Rückstand erhält man ein gelbes, viskoses Öl.

Das vorhergehend erhaltene Öl wird in 50 ml entionisiertem Wasser gelöst und mit ca. 20 ml Wasser in einen mit Stickstoff begasten Kutscher-Steudel-Extraktor gespült. Hierauf wird das Gemisch mit etwa 500 ml n-Hexan während 20 Stunden kontinuierlich extrahiert. Nach beendeter Extraktion werden die beiden im Extraktor verbleibenden Phasen in einem Scheidetrichter getrennt. Die organischen Phasen werden vereinigt, über etwa 30 g $Na_2SO_4$ getrocknet und am Rollverdampfer bei 45°C/100 - 20 mbar eingedampft. Zurück bleiben 17,5 g gelbes Trihexylamin mit einem Gehalt von 99,0 %. Dies entspricht einer Ausbeute an Trihexylamin von 99 6 %.

Die nach der Extraktion des Trihexylamins angefallene Wasserphase enthält 21,2 g Ascorbinsäure, entsprechend einer Ausbeute von 93,5 %.

**Beispiel 4**

In einen 100-ml-Sulfierkolben, versehen mit Rührer, Thermometer, Rückflußkühler und 50-ml-Tropftrichter, werden unter Argon 26,8 g (128,7 mMol) Ketogulonsäuremethylester und 10 ml Methanol gegeben. Hierauf wird das Gemisch unter Rühren auf 65°C (Rückfluß) erwärmt. Mittels des Tropftrichters werden dann 34,7 g (100 Mol-%) Trihexylamin (99,9 %-ig) innert 15 Minuten zugetropft. Nach ungefähr 4 Stunden geht alles in Lösung, das Reaktionsgemisch bleibt jedoch bis zum Schluß trübe. Während 4 3/4 Stunden wird bei Rückfluß weitergerührt, wobei sich das Reaktionsgemisch gelb färbt. Nach insgesamt 5 Stunden wird das Gemisch mit Methanol in einen 500-ml-Rundkolben gespült und am Rollverdampfer bei 45°C/20 mbar eingeengt. Als Rückstand erhält man ein gelbes, viskoses Öl.

Das vorhergehend erhaltene Öl wird in 50 ml entionisiertem Wasser gelöst und mit ca. 20 ml Wasser in einen mit Stickstoff begasten Rotations-Perforator gespült. Hierauf wird das Gemisch mit etwa 500 ml n-Hexan während 20 Stunden kontinuierlich extrahiert. Nach beendeter Extraktion werden die beiden im Extraktor verbleibenden Phasen in einem Scheidetrichter getrennt. Die organischen Phasen werden vereinigt, über etwa 30 g $Na_2SO_4$ getrocknet und am Rollverdampfer bei 45°C/100 - 20 mbar eingedampft. Zurück bleiben 34,4 g gelbes Trihexylamin mit einem Gehalt von 98,3 %. Dies entspricht einer Ausbeute an Trihexylamin von 97,5 %.

Die nach der Extraktion des Trihexylamins angefallene Wasserphase enthält 21,0 g Ascorbinsäure, entsprechend einer Ausbeute von 92,7 %.

**Beispiel 5**

In einen 500-ml-Sulfierkolben, versehen mit Rührer, Thermometer, Rückflußkühler und 50-ml-Tropftrichter, werden unter Argon 26,8 g (128,7 mMol) Ketogulonsäuremethylester und 125 ml Methanol gegeben. Hierauf wird das Gemisch unter Rühren auf 65°C (Rückfluß) erwärmt, wobei sich der Ester löst. Durch den Tropftrichter werden dann 33,1 g (106,5 Mol-%) Bis-(2-ethylhexyl)-amin (100 %-ig) unter Aufrechterhaltung eines pH-Wertes von etwa 6,5 während 40 Minuten zugegeben. Anschließend wird noch 3 1/2 Stunden bei Rückfluß nachreagiert, wobei das Reaktionsgemisch gelb wird. Dann wird am Rollverdampfer bei 45°C/20 mbar das Methanol abdestilliert. Zurück bleibt gelbes, kristallines Ascorbinsäure-Aminsalz.

Die vorhergehend erhaltenen Kristalle werden mit 400 ml Wasser und 400 ml n-Hexan aufgeschlämmt und eine Stunde lang kräftig gerührt, wobei sich das Salz vollständig auflöst. Hierauf wird die Wasserphase mit 2 x 100 ml n-Hexan extrahiert. Die Hexanphasen werden mit 2 x 100 ml Wasser zurückgewaschen, über etwa 40 g

$Na_2SO_4$ getrocknet und am Rollverdampfer bei 45° C/100 - 20 mbar eingedampft. Zurück bleiben 29,2 g (86,5 %) hellgelbes Bis-(2-ethylhexyl)-amin mit einem Gehalt von 98 %.

Die vereinigten Wasserphasen werden auf ca. 100 ml eingeengt und in einem Kutscher-Steudel-Extraktor während 20 Stunden mit etwa 300 ml n-Hexan kontinuierlich nachextrahiert. Die organische Phase wird über ca. 20 g $Na_2SO_4$ getrocknet und am Rollverdampfer bei 45° C/100-20 mbar eingedampft. Man erhält so noch 3,55 g (10,3 %) gelbes Bis-(2-ethylhexyl)-amin mit einem Gehalt von 96,1 %. Dies entspricht einer Totalausbeute an Amin von 96,8 %.

Die aus der Extraktion des Amins angefallenen, vereinten Wasserphasen enthalten 21,3 g Ascorbinsäure, entsprechend einer Ausbeute von 94,1 %.

**Beispiel 6**

In einen 200-ml-Sulfierkolben, versehen mit Rührer, Thermometer, Rückflußkühler und 500-ml-Tropftrichter, werden unter Argon 26,8 g (128,7 mMol) Ketogulonsäuremethylester und 25 ml Methanol gegeben. Hierauf wird das Gemisch unter Rühren auf 65° C (Rückfluß) erwärmt. Mittels des Tropftrichters werden dann 29,8 g (100 Mol-%) Tripentylamin (98,3 %-ig) innert 15 Minuten zugetropft. Nach ungefähr einer Stunde geht alles in Lösung über. Während 4 3/4 Stunden wird bei Rückfluß weitergerührt, wobei sich das Reaktionsgemisch gelb färbt. Nach insgesamt 5 Stunden wird das Gemisch mit Methanol in einen 500-ml-Rundkolben gespült und am Rollverdampfer bei 45° C/20 mbar eingeengt. Als Rückstand erhält man ein gelbes, viskoses Öl.

Das vorhergehend erhaltene Öl wird in 50 ml entionisiertem Wasser gelöst und mit ca. 20 ml Wasser in einen mit Stickstoff begasten Rotations-Perforator gespült. Hierauf wird das Gemisch mit etwa 500 ml n-Hexan während 20 Stunden kontinuierlich extrahiert. Nach beendeter Extraktion werden die beiden im Extraktor verbleibenden Phasen in einem Scheidetrichter getrennt. Die organischen Phasen werden vereinigt, über etwa 30 g $Na_2SO_4$ getrocknet und am Rollverdampfer bei 45° C/100 - 20 mbar eingedampft. Zurück bleiben 29,3 g gelbes Tripentylamin mit einem Gehalt von 98,7 %. Dies entspricht einer Ausbeute an Tripentylamin von 98,9 %.

Die nach der Extraktion des Tripentylamins angefallene Wasserphase enthält 21,9 g Ascorbinsäure, entsprechend einer Ausbeute von 96,5 %.

**Beispiel 7**

In zu den Beispielen 1 bis 6 analoger Weise wurden weitere Amine verwendet. Die Resultate sind in der folgenden Tabelle zusammengestellt.

**Tabelle**

| Amin | Ausbeute an Ascorbinsäure | Zurückgewonnenes Amin |
|---|---|---|
| Primene® JMT | 95,4 % | 79,2 % |
| α-Amino-diphenylmethan | 90,8 % | 66,8 % |
| 1,2-Diphenyläthylamin | 89,2 % | 49,8 % |
| Amberlite® LA-3[a] | 97,0 % | 56,5 % |
| Dibenzylamin | 91,9 % | 83,3 % |
| Dioctylamin | 96,2 % | 94,3 % |
| Triisopentylamin | 94,3 % | 94,9 % |
| N,N-Dioctylmethylamin | 92,7 % | 91,3 % |
| Triheptylamin | 95,8 % | 100 % |
| Tridodecylamin | 90,4 % | 96,1 % |
| Amberlite® LA-1 [a] | 88,3 % | 96,5 % |
| Amberlite® LA-2 [a] | 85,6 % | 94,3 % |

[a] Flüssiger Ionentauscher der Firma Rohm u. Haas.

**Beispiel 8**

In einen 500-ml-Sulfierkolben, versehen mit Rührer, Thermometer Rückflußkühler und 100-ml-Tropftrichter, werden unter Argon 26,8 g (128,7 mMol) Ketogulonsäuremethylester und 125 ml Methanol gegeben. Hierauf

wird das Gemisch unter Rühren auf 65°C (Rückfluß) erwärmt, wobei sich der Ester löst. Mittels des Tropftrichters werden dann 46,2 g (100 Mol-%) Trioctylamin innert 15 Minuten zugetropft. Nun wird während 4 3/4 Stunden bei Rückfluß weitergerührt, wobei sich das Reaktionsgemisch gelb färbt. Nach insgesamt 5 Stunden wird das Gemisch am Rollverdampfer bei 45°C/20 mbar eingeengt, wobei ein viskoses zweiphasiges Öl zurückbleibt.

Das vorhergehend erhaltene Öl wird in 150 ml Toluol gelöst und unter Rühren auf Rückflußtemperatur erwärmt. Beim Aufheizen beginnt schon ziemlich rasch weiße Ascorbinsäure zu kristallisieren. Anschließend wird noch 1 Stunde bei Rückflußtemperatur weitergerührt und der Niederschlag dann heiß abgenutscht. Der Rückstand wird mit 15 ml Toluol und dann mit 15 ml Hexan gewaschen und über Nacht bei Raum-temperatur/20 mbar im Vakuumtrockenschrank getrocknet. Man erhält 20,75 g (91,5 %) reine Ascorbinsäure.

Das bei der Filtration anfallende Filtrat wird am Rotationsverdampfer vollständig eingedampft und man erhält 50,8 g (110 %) rohes Trioctylamin zurück.

**Beispiel 9**

In einen 500-ml-Sulfierkolben, versehen mit Rührer, Thermometer, Rückflusskühler und 50-ml-Tropftrichter, werden unter Argon 26,8 g (128,7 mMol) Ketogulonsäuremethylester und 125 ml Methanol gegeben. Hierauf wird das Gemisch unter Rühren auf 65°C (Rückfluss) erwärmt, wobei sich der Ester löst. Mittels des Tropftrichters werden dann 46,2 g (100 Mol-%) Trioctylamin (98,3 %-ig) innert 15 Minuten zugetropft. Nun wird währerd4 3/4 Stunden bei Rückfluss weitergerührt, wobei sich das Reaktionsgemisch gelb färbt. Nach insgesamt 5 Stunden wird das Gemisch mit Methanol in einen 500-ml-Rundkolben gespült und am Rollverdampfer bei 45°C/20 mbar eingeengt. Als Rückstand erhält man ein viskoses, zweiphasiges Öl.

Das vorhergehend erhaltene Öl wird mit 150 ml Hexan versetzt und unter Rühren auf Rückflusstemperatur (57°C) erwärmt. Ziemlich rasch beginnt gelbliche Ascorbinsäure zu kristallisieren. Nach 1 1/2 Stunden bei Rückfluss werden die Kristalle heiss abgenutscht, mit 2 x 15 ml Hexan gewaschen und über Nachtbei Raumtemperatur/20 mbar im Vakuumtrockenschrank getrocknet. Man erhält 23,6 g Ascorbinsäure mit einem Gehalt von 90,6 %. (Ausbeute 94,3 %).

Das bei der Filtration anfallende Filtrat wird am Rotationsverdampfer vollständig eingedampft und man erhält 44,8 g 97,1 %-iges Trioctylamin zurück. Dies entspricht einer Ausbeute von 95,7 %.

**Beispiel 10**

In einen 500-ml-Sulfierkolben, versehen mit Rührer, Thermometer, Rückflußkühler und 50-ml-Tropftrichter, werden unter Argon 30,4 g (128,7 mMol) Ketogulonsäureisopropylester und 125 ml Methanol gegeben, wobei sich der Ester löst. Hierauf wird die Lösung unter Rühren auf 65°C (Rückfluß) erwärmt. Mittels des Tropftrichters werden dann 34,7 g (100 Mol-%) Trihexylamin (99,9 %-ig) innert 15 Minuten zugetropft. Gegen Ende der Trihexylamin-Zugabe wird das Reaktionsgemisch leicht trübe, 5 Minuten später klärt sich die Lösung jedoch wieder. Nun wird während 6 3/4 Stunden bei Rückfluß weitergerührt, wobei sich das Reaktionsgemisch gelb färbt. Nach insgesamt 7 Stunden wird das Gemisch mit Methanol in einen 500-ml-Rundkolben gespült und am Rollverdampfer bei 45°C/20 mbar eingeengt. Als Rückstand erhält man ein gelbes, viskoses Öl.

Das vorhergehend erhaltene Öl wird in 50 ml entionisiertem Wasser gelöst und mit ca. 20 ml Wasser in einen mit Stickstoff begasten Rotations-Perforator gespült. Hierauf wird das Gemisch mit etwa 500 ml n-Hexan während 20 Stunden kontinuierlich extrahiert. Nach beendeter Extraktion werden die beiden im Extraktor verbleibenden Phasen in einem Scheidetrichter getrennt. Die organischen Phasen werden vereinigt, über etwa 30 g $Na_2SO_4$ getrocknet und am Rollverdampfer bei 45°C/100 - 20 mbar eingedampft. Zurück bleiben 36,4 g gelbes Trihexylamin mit einem Gehalt von 95,6 %. Dies entspricht einer Ausbeute an Trihexylamin von 100 %.

Die nach der Extraktion des Trihexylamins angefallene Wasserphase enthält 16,1 g Ascorbinsäure, entsprechend einer Ausbeute von 70,8 %.

**Beispiel 11**

In einen 750-ml-Sulfierkolben, versehen mit Rührer, Thermometer, Rückflußkühler und 250-ml-Tropftrichter, werden unter Argon 111,1 g Ketogulonsäureäthylester (Gehalt: 91 %) und 100 ml Äthanol gegeben. Hierauf wird das Gemisch unter Rühren auf 65°C erwärmt, wobei sich der Ester löst. Mittels des Tropftrichters werden dann 134,8 g Trihexylamin (99,9 %-ig) innert 25 Minuten zugetropft. Nun wird während 4 1/2 Stunden bei 65°C weitergerührt, wobei sich das Reaktionsgemisch rot-braun färbt. Nach insgesamt 5 Stunden wird das Gemisch mit Äthanol in einen 1000-ml-Rundkolben gespült und am Rollverdampfer bei 45°C/20 mbar eingeengt. Als Rückstand erhält man ein braunes, viskoses Öl.

Das vorhergehend erhaltene Öl wird in 250 ml entionisiertem Wasser gelöst und 2 x mit 150 ml n-Hexan

extrahiert. Die Hexanphasen werden mit 50 ml Wasser nachgewaschen und die vereinigten Wasserphasen in einem Rotations-Perforator mit etwa 800 ml n-Hexan während 20 Stunden kontinuierlich nachextrahiert. Nach beendeter Extraktion werden die beiden im Extraktor verbleibenden Phasen in einem Scheidetrichter getrennt. Die organischen Phasen werden vereinigt, über etwa 50 g $Na_2SO_4$ getrocknet und am Rollverdampfer bei 45°C/100 - 20 mbar eingedampft. Zurück bleiben 136,7 g gelbes Trihexylamin mit einem Gehalt von 98,6 %. Dies entspricht einer Ausbeute an Trihexylamin von 100 %.

Die nach der Extraktion des Trihexylamins angefallene Wasserphase enthält 63,1 g Ascorbinsäure, entsprechend einer Ausbeute von 78,5 % und 14,8 g Ketogulonsäureäthylester entsprechend einer Ausbeute von 14,7 %.

**Beispiel 12**

In zu Beispiel 1 analoger Weise wurde die Umsetzung des Ketogulonsäuremethylesters mit Trihexylamin in Methanol durchgeführt; die Extraktion wurde jedoch mit Petroläther bzw. Toluol durchgeführt. Die Resultate sind nachfolgend zusammengefaßt:

| Extraktionsmittel | Ausbeute an Ascorbinsäure | zurückgewonnenes Amin |
|---|---|---|
| Toluol | 93,9 % | 97,4 % |
| Petroläther | 98,5 % | 100,0 % |
| Methylcyclohexan | 97,2 % | 99,5 % |

**Beispiel 13**

In einen 500-ml-Sulfierkolben, versehen mit Rührer, Thermometer, Rückflußkühler und 50-ml-Tropftrichter, werden unter Argon 26 8 g (128,7 mMol) Ketogulonsauremethylester und 125 ml Acetonitril gegeben. Hierauf wird das Gemisch unter Rühren auf 78°C (Rückfluß) erwärmt. Mittels des Tropftrichters werden dann 34,7 g (100 Mol-%) Trihexylamin (99,4 %-ig) innert 15 Minuten zugetropft. Nach ~1 Stunde Reaktionszeit geht der Ester vollständig in Lösung. Nachdem insgesamt 2 1/2 Stunden bei Rückflußtemperatur gerührt wurde, wird das orange-braune Reaktionsgemisch in einen mit Stickstoff begasten Rotations-Perforator gegeben. Hierauf wird das Gemisch mit etwa 500 ml n-Hexan während 20 Stunden kontinuierlich extrahiert. Schon 1 Stunde nach Extraktionsbeginn, kristallisiert aus der Acetonitrilphase langsam Ascorbinsäure aus. Nach beendeter Extraktion wird der Inhalt des Extraktors abgenutscht. Die gelblichen Kristalle werden mit Hexan und Acetonitril gewaschen und im Exsiccator über Nacht getrocknet. Man erhält 20,6 g Ascorbinsäure mit einem Gehalt von 99,5 %, entsprechend einer Ausbeute von 90,4 %.

Die bei der Filtration angefallene Mutterlauge wird im Scheidetrichter getrennt. Die beiden Hexanphasen werden vereinigt, über etwa 30 g $Na_2SO_4$ getrocknet und am Rollverdampfer bei 45°C/100 - 20 mbar eingedampft. Zurück bleiben 34,9 g gelbes Trihexylamin mit einem Gehalt von 98,2 %. Dies entspricht einer Ausbeute an Trihexylamin von 99,4 %.

Die orange Acetonitrilphase enthält noch 0,8 g Ascorbinsäure. Zusammen mit der kristallisierten Ascorbinsäure entspricht dies einer Ausbeute von 93,7 %.

**Patentansprüche**

1. Verfahren zur Herstellung von Ascorbinsäure durch Umlagerung von Ketogulonsäureestern in alkalischem Milieu, dadurch gekennzeichnet, daß man einen Ketogulonsäureester der allgemeinen Formel

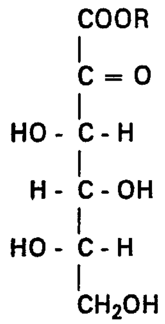

I

worin R $C_{1-5}$-Alkyl bedeutet,

mit einem Amin mit 12 bis 38 Kohlenstoffatomen in einem protischen oder aprotisch-dipolaren organischen Lösungsmittel umsetzt,und daß man das erhaltene Ascorbinsäure-Aminsalz der allgemeinen Formel

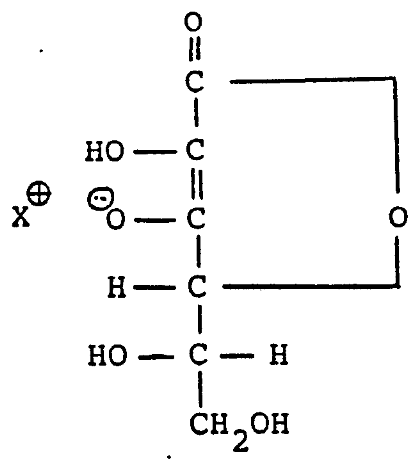

II

worin $X^{\oplus}$ das Ammoniumion des eingesetzten Amins darstellt,

ohne Neutralisation spaltet und die Ascorbinsäure sowie gegebenenfalls das eingesetzte Amin isoliert, wobei die Isolierung der ersteren durch flüßig-flüßig Extraktion oder durch Digerieren in einem unpolaren organischen Lösungsmittel erfolgt.

2. Verfahren nach Anspruch 1, dadurch gekennzeichnet, daß man als Amin ein geradkettiges tertiäres Alkylamin mit 15 bis 30 Kohlenstoffatomen verwendet.

3. Verfahren nach Anspruch 1, dadurch gekennzeichnet, daß man als Amin ein verzweigtes sekundäres Alkylamin mit 16 bis 25 Kohlenstoffatomen verwendet.

4. Verfahren nach Anspruch 1, dadurch gekennzeichnet, daß man als Amin ein verzweigtes primäres Alkylamin mit 12 bis 24 Kohlenstoffatomen verwendet..

5. Verfahren nach Anspruch 1 oder 2, dadurch gekennzeichnet, daß man als Amin Tripentylamin, Triisopentylamin, N,N-Dioctylmethylamin, Trihexylamin, Triheptylamin, Trioctylamin oder Tridodecylamin verwendet.

6. Verfahren nach Anspruch 1 oder 3, dadurch gekennzeichnet, daß man als Amin Bis-(2-äthyl-hexyl)-amin, Dibenzylamin, Dioctylamin, Amberlite® LA-1 oder Amberlite® LA-2 verwendet.

7. Verfahren nach Anspruch 1 oder 4, dadurch gekennzeichnet, daß man als Amin Primene® JMT, Amberlite® LA-3, α-Amino-diphenylmethan oder 1,2-Diphenyläthylamin verwendet.

8. Verfahren nach einem der Ansprüche 1 bis 7, dadurch gekennzeichnet, daß man als organisches Lösungsmittel einen niederen Alkohol mit 1 bis 5 Kohlenstoffatomen und vorzugsweise Methanol verwendet.

9. Verfahren nach einem der Ansprüche 1 bis 8, dadurch gekennzeichnet, daß das Amin in einer Menge von 0,1 bis 1,5 pro Mol Ketogulonsäureester verwendet wird.

10. Verfahren nach Anspruch 9, dadurch gekennzeichnet, daß das Amin in einer Menge von 0,5 bis 1,1 pro Mol Ketogulonsäureester verwendet wird.

11. Verfahren nach Anspruch 9, dadurch gekennzeichnet, daß das Amin in einer Menge von 0,9 bis 1 Mol pro Mol Ketogulonsäureester verwendet wird.

12. Verfahren nach einem der Ansprüche 1 bis 10, dadurch gekennzeichnet, daß man die Umsetzung des Ketogulonsäureesters mit dem Amin bei einer Temperatur bis 90°C durchführt.

13. Verfahren nach Anspruch 12, dadurch gekennzeichnet, daß man die Umsetzung des Ketogulonsäureesters mit dem Amin bei einer Temperatur von 50°C bis 75°C durchführt.

14. Verfahren nach Anspruch 13, dadurch gekennzeichnet, daß man die Umsetzung des Ketogulonsäureesters mit dem Amin bei einer Temperatur von 65°C durchführt.

**Claims**

1. A process for the manufacture of ascorbic acid by rearranging ketogulonic acid esters in an alkaline medium, characterized by reacting a ketogulonic acid ester of the general formula

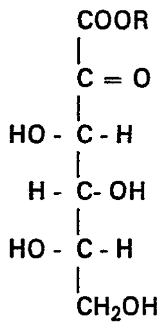

I

wherein R signifies $C_{1-5}$-alkyl,

with an amine having 12 to 38 carbon atoms in a protic or aprotic-dipolar organic solvent and cleaving the resulting ascorbic acid amine salt of the general formula

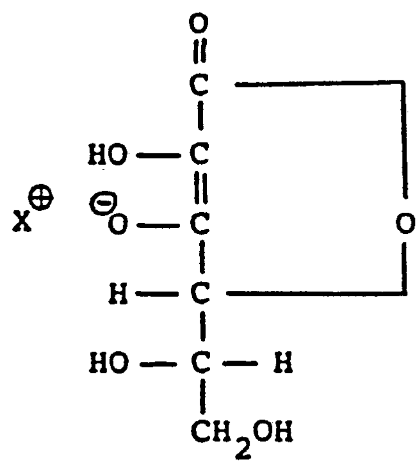

II

wherein $X^{\oplus}$ represents the ammonium ion of the amine used,

without neutralization and isolating the ascorbic acid as well as, if desired, the amine used, whereby the isolation of the former is effected by liquid-liquid extraction or by digestion in a non-polar organic solvent.

2. A process according to claim 1, characterized in that a straight-chain tertiary alkylamine having 15 to 30 carbon atoms is used as the amine.

3. A process according to claim 1, characterized in that a branched secondary alkylamine having 16 to 25 carbon atoms is used as the amine.

4. A process according to claim 1, characterized in that a branched primary alkylamine having 12 to 24 carbon atoms is used as the amine.

5. A process according to claim 1 or 2, characterized in that tripentylamine, triisopentylamine, N,N-dioctylmethylamine, trihexylamine, triheptylamine, trioctylamine or tridodecylamine is used as the amine.

6. A process according to claim 1 or 3, characterized in that bis-(2-ethyl-hexyl)amine, dibenzylamine, dioctylamine, Amberlite® LA-1 or Amberlite® LA-2 is used as the amine.

7. A process according to claim 1 or 4, characterized in that Primen® JMT, Amberlite® LA-3, αaminodiphenylmethane or 1,2-diphenylethylamine is used as the amine.

8. A process according to any one of claims 1 to 7, characterized in that a lower alcohol having 1 to 5 carbon atoms, preferably methanol, is used as the organic solvent.

9. A process according to any one of claims 1 to 8, characterized in that the amine is used in an amount of 0.1 to 1.5 per mol of ketogulonic acid ester.

10. A process according to claim 9, characterized in that the amine is used in an amount of 0.5 to 1.1 per mol of ketogulonic acid ester.

11. A process according to claim 9, characterized in that the amine is used in an amount of 0.9 to 1 mol per mol of ketogulonic acid ester.

12. A process according to any one of claims 1 to 10, characterized in that the reaction of the ketogulonic acid ester with the amine is carried out at a temperature up to 90° C.

13. A process according to claim 12, characterized in that the reaction of the ketogulonic acid ester with the

amine is carried out at a temperature of 50° C to 75° C.

14. A process according to claim 13, characterized in that the reaction of the ketogulonic acid ester with the amine is carried out at a temperature of 65° C.

## Revendications

1. Procédé de préparation de l'acide ascorbique par transposition d'esters cétoguloniques en milieu alcalin, caractérisé en ce que l'on fait réagir un ester cétogulonique de formule générale

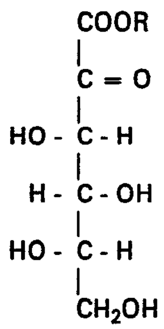

I

dans laquelle R représente un groupe alkyle en $C_1$-$C_5$, avec une amine contenant 12 à 38 atomes de carbone dans un solvant organique protonique ou aprotonique-dipolaire et en ce que l'on scinde le sel d'amine de l'acide ascorbique ainsi obtenu, répondant à la formule générale

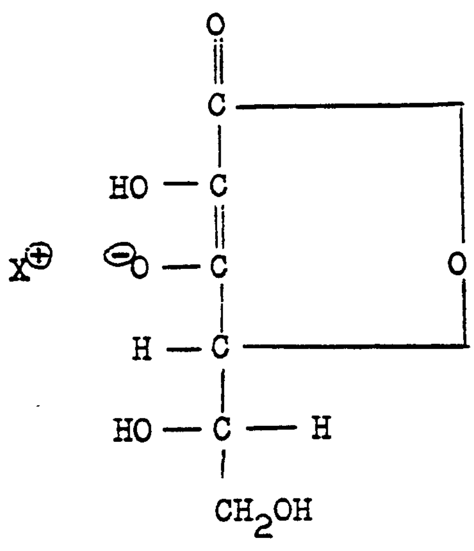

II

dans laquelle $X^{\oplus}$ représente l'ion ammonium correspondant à l'amine mise en oeuvre, sans le neutraliser, et on isole l'acide ascorbique et le cas échéant l'amime mise en oeuvre, l'isolement du premier étant réalisé par extraction liquide-liquide ou par digestion dans um solvamt organique non polaire.

2. Procédé selom la revendication 1, caractérisé en ce que l'amine utilisée est une alkylamine tertiaire à chaîne droite contenant 15 à 30 atomes de carbone.

3. Procédé selon la revendication 1 caractérisé en ce que l'amine utilisée est une alkylamine secondaire ramifiée contenant 18 à 25 atomes de carbone.

4. Procédé selon la revendication 1, caractérisé en ce que l'amine utilisée est une alkylamine primaire ramifiée contenant 12 à 24 atomes de carbone.

5. Procédé selon la revendication 1 ou 2, caractérisé en ce que l'amine utilisée est la tripentylamine, la triisopentylamine, la N,N-dioctylméthylamine, la trihexylamine, la triheptylamine, la trioctylamine ou la tridodécylamine.

6. Procédé selon la revendication 1 ou 3, caractérisé en ce que l'amine utilisée est la bis-(2-éthylhexyl)-amine, la dibenzylamine, la dioctylamine, ou la résine du commerce Amberlite® LA-1 ou Amberlite® LA-2.

7. Procédé selon la revendication 1 ou 4, caractérisé en ce que l'amine utilisée est l'échangeur d'ions du commerce Primene® JMT, la résine du commerce Amberlite® LA-3, l'α-amino-diphénylméthane ou la 1,2-diphényléthylamine.

8. Procédé selon l'une des revendications 1 à 7, caractérisé en ce que le solvant organique utilise est un alcool inférieur en $C_1$-$C_5$, de préférence le méthanol.

9. Procédé selon l'une des revendications 1 à 8, caractérisé en ce que l'on utilise l'amine en quantité de 0,1 à 1,5 mole par mole d'ester cétogulonique.

10. Procédé selon la revendication 9, caractérisé en ce que l'on utilise l'amine en quantité de 0,5 à 1,1 mole par mole d'ester cétogulonique.

11. Procédé selon la revendication 9, caractérisé en c e que l'on utilise l'amine en quantité de 0,9 à 1 mole par mole d'ester cétogulonique.

12. Procédé selon l'une des revendications 1 à 10, caractérisé en ce que la réaction entre l'ester cétogulonique et l'amine est effectuée à une température allant jusqu'à 90° C.

13. Procédé selon la revendication 12, caractérisé en ce que la réaction entre l'ester cétogulonique et l'amine est effectuée à une température de 50 à 75° C.

14. Procédé selon la revendication 13, caractérisé en ce que la réaction entre l'ester cétogulonique et l'amine est effectuée à une température de 65° C.